# EUROPEAN PATENT SPECIFICATION

(11) **EP 4 688 708 B1**
(45) Date of publication and mention of the grant of the patent: **26.08.2026**
(21) Application number: 24716124.3
(22) Date of filing: 27.03.2024
(51) Int. Cl.: C07C 4/22, C08J 11/12, C10G 1/10

(54) **DEPOLYMERIZATION OF CROSS-LINKED POLYETHYLENE**
DEPOLYMERISIERUNG VON VERNETZTEM POLYETHYLEN
DÉPOLYMÉRISATION DE POLYÉTHYLÈNE RÉTICULÉ

(30) Priority: 30.03.2023 EP 23165471
(43) Date of publication of application: 11.02.2026
(73) Proprietor: Basell Poliolefine Italia S.r.l., 20121 Milano (IT)
(72) Inventor: MIHAN, Shahram, 65926 Frankfurt/M. (DE); FRAAIJE, Volker, 65926 Frankfurt/M. (DE); MANNEBACH, Gerd, 65926 Frankfurt/M. (DE); BRÜNING, Holger, 65926 Frankfurt/M. (DE); JUSTUS, Christof, 65926 Frankfurt/M. (DE); LEIBOLD, Hans, 76227 Karlsruhe (DE); RICHTER, Frank, 66386 St. Ingbert (DE)
(74) Representative: LyondellBasell
(86) International application number: PCT/EP2024/058263
(87) International publication number: WO 2024/200518

(56) References cited:
- WO-A1-2018/216031
- WO-A1-2023/011875
- DE-A1- 19 822 568
- MARCILLA, A. ET AL: "Thermal and catalytic pyrolysis of crosslinked polyethylene", JOURNAL OF ANALYTICAL AND APPLIED PYROLYSIS ELSEVIER BV, NL, vol. 76, no. 1-2, 1 June 2006 (2006-06-01), pages 254 - 259, XP024998782, ISSN: 0165-2370, [retrieved on 20060601], DOI: 10.1016/J.JAAP.2005.12.004

## Description

### FIELD OF THE DISCLOSURE

The present disclosure refers to a method for the production of a cracker feedstock by depolymerization of cross-linked polyethylene and/or multicomponent materials comprising polyolefins.

### BACKGROUND OF THE DISCLOSURE

Cross-linked polyethylene, commonly abbreviated PEX, XPE or XLPE, is predominately used in multicomponent materials, such as in building service pipework systems, hydronic radiant heating or floor heating and cooling systems, domestic water piping, and insulation for high tension electrical cables. Cross-linked polyethylene can further be found in natural gas and offshore oil applications, district heating, chemical transportation, and transportation of sewage and slurries, recently also in mining.

The benefits of using cross-linked polyethylene are its flexibility, its low costs and easier installation than, e.g., copper pipes. Cross-linked polyethylene also shows an improved longevity as it is not non-corrodible, extreme high stress crack and abrasion resistance and thus believed to be a suitable candidate for progressive replacement of metal and thermoplastic pipes.

Although the expected lifetime of PEX pipes is expected to reach 50 years, replacement will at one point become necessary, especially as the end of the first generation of pipe materials which were installed 40 to 50 years ago is rapidly approaching. However, up to date, recycling of cross-linked polyethylene is still a challenge as it may be shredded and compounded together with non-crosslinked material, the thus obtained material cannot be used in standard applications, since the ultra-high molecular weight of the crosslinked polymer chains prevents a homogenous mixing and causes the formation of large gels. Mechanical recycling which is the standard procedure for recycling of plastics is therefore not an option.

WO 2018/216031 refers to a system and method for re-conversion of plastics into different petrochemical liquid and gaseous hydrocarbons. The system is mainly comprised of a reactor, a burning chamber, a feeding unit and a gas collection and cooling unit. For the re-conversion method, the plastic material in granular form along with 2% manganese sulfate as a catalyst is loaded into the reactor and heated to first depolymerize and then form a gaseous mixture of petrochemical compounds.

DE 198 22 568 suggests a process for recycling plastic which is also supposed to be applicable for crosslinked polyethylene in which plastic is brought into contact with a catalyst at elevated temperature and a resulting gaseous phase of the reaction product is at least partially condensed.

US 2017/0327663 discloses a system for continuously treating recycled polymeric material comprising (a) a hopper configured to continuously feed said recycled polymeric material into said system, (b) an extruder system configured to continuously turn said recycled polymeric material in a molten state; (c) a filter system configured to continuously filter said molten material, (d) a first reactor configured to continuously depolymerize said molten material into a depolymerized molten material; (e) a heat exchanger configured to continuously cool said molten depolymerized material; and (f) a purifier system configured to continuously purify said molten material.

The first processes for recycling of crosslinked polyethylene were limited to a laboratory set-up or the crosslinked polyethylene could only be used for the production of various waxes after depolymerization. There is therefore still the need for an efficient recycling process for crosslinked polyethylene material which aims to return the waste material to its molecular form for use as a feedstock for new plastic material.

This need is addressed by the present disclosure which provides a method for the production of a cracker feedstock by depolymerization of crosslinked polyethylene.

### SUMMARY OF THE DISCLOSURE

In a first embodiment, the present disclosure thus presents a method for the production of a cracker feedstock by depolymerization of polymeric waste material comprising crosslinked polyethylene, the method comprising the steps of
i) introducing the waste material feedstock into a pyrolysis reactor, preferably a reactor with a screw conveyer;
ii) mixing the waste material with a depolymerization catalyst;
iii) depolymerizing the waste material in an oxygen-free atmosphere to obtain a gaseous depolymerization product;
iv) collecting and condensing at least part of the gaseous depolymerization product to obtain the desired cracker feedstock.

In a preferred embodiment the catalyst is a particulate catalyst comprising an acidic compound as the active component, the acidic compound of the catalyst being preferably deposited on a particulate non-porous support preferably with the aid of a coating agent.

In a preferred embodiment, the particulate non-porous support is selected from the group consisting of sand, glass beads and metal particles.

In a preferred embodiment, the coating agent is selected from the group consisting of oil, inorganic hydrogel and combinations thereof.
In a preferred embodiment, the acidic compound is selected from the group consisting of Al/Si mixed oxides, Al₂O₃, aluminosilicates, silica and zeolites.

In one embodiment, the waste material further comprises aluminum and in that the method further comprises a step of collecting the aluminum from the waste material.

In a preferred embodiment, the crosslinked polyethylene in the polymeric waste material is selected from the group consisting of peroxide-crosslinked polyethylene, silane-crosslinked polyethylene, irradiation-crosslinked polyethylene, radically crosslinked polyethylene, azo-crosslinked polyethylene, UV initiated radical crosslinked polyethylene and mixtures thereof.

In a preferred embodiment, the waste material is obtained from PEX pipes, PEX/Al/PEX pipes, Al/PEX pipes and/or PEX/Al/PEX pipes.

In a preferred embodiment, the polymeric waste material comprises or consists of shredded PEX pipe waste and plastic waste stream with a melt index (MFI, 21.6) of higher than 10 g/10 min, with preference given to an amount of plastic waste stream of at least 40 wt.-% of the waste material, preferably 40 to 80 wt.-%, based on the total weight of the waste material.

The polymeric waste material according to the present disclosure preferably contains in the in the polymeric fraction of the waste material, a low amount of non-polyolefin components and in particular an amount lower than 12 wt.%, and even more preferably less than 10 wt.% of the total weight of the dry weight polymeric fraction of the waste material feedstock.

In a preferred embodiment, the gaseous depolymerization product is released from the reactor by gas release units placed along a screw conveyer.

In a preferred embodiment, the collection of the gaseous depolymerization product comprises a step of hot gas filtration.

In a preferred embodiment, condensing of the gaseous depolymerization product is achieved by sequentially passing the gaseous depolymerization product through a plurality of condensing units.

In a preferred embodiment, the residence time of the waste material in the reactor is no more than 60 minutes, preferably no more than 45 minutes.

In a preferred embodiment, the reactor is operated at a temperature of 350 to 650 °C, preferably 400 to 590°C more preferably from 430 to 500°C.

In a preferred embodiment, the reactor is operated at a pressure of 0.7 to 10 bar_{g}.

In one embodiment, the reactor is heated electrically.

In a preferred embodiment, at least part of any non-liquefiable depolymerization product and/or at least part of the catalyst is re-introduced into the reactor.

In a preferred embodiment, the method further comprises monitoring the content of methane in the collected gaseous depolymerization product.

In a preferred embodiment, the collected depolymerization product comprises more than 60 wt.-% of gaseous components, based on the total weight of the depolymerization product with the gaseous components preferably comprising more than 50 wt.-% of olefins, based on the total weight of the gaseous components.

In a preferred embodiment, the liquid depolymerization product comprises from 35 to 45 wt.-% of a high boiling fraction, from 40 to 50 wt.-% of a medium boiling fraction, and from 15 to 25 wt.-% of a low boiling fraction.

### DETAILED DESCRIPTION OF THE DISCLOSURE

In one aspect the present disclosure refers to a method for the production of a cracker feedstock by depolymerization of polymeric waste material comprising crosslinked polyethylene using an acidic depolymerization catalyst.

In a first step of the method according to the present disclosure, the polymeric waste material feedstock is introduced into a pyrolysis reactor, preferably a reactor with a screw conveyer. The use of the screw conveyer was found to be especially useful in combination with the particulate catalyst. This way a homogenous mixture of waste material feedstock and catalyst was obtained thereby achieving a homogeneous heating of the waste material feedstock with the catalyst to be believed to act as heat carrier while at the time preventing the molten waste material feedstock from sticking to the screw.

In order to assist in an efficient pyrolysis and depolymerization of the polymeric waste material, the catalyst of the present disclosure is preferably in particulate form. Preference is given to particulate non-porous support selected from the group consisting of sand, glass beads and metal particles. The particulate non-porous support may have any shape such as spherical, cylindrical or any non-homogenous shape. Apart from being particulate, the support employed in the catalyst of the present invention is also non-porous. Non-porous within the meaning of the present disclosure is to be understood as being not permeable to air, water or other liquids.

The catalyst of the present disclosure is in particular designed to be mixed with the polymeric waste material undergoing depolymerization. To ensure sufficient mixing, the catalyst preferably has an average particle size of 0.2 to 20 mm, preferably 0.5 to 10 mm, determined according to Coulter counter analysis in accordance with ASTM D4438. Non-porous particulate support, preferably sand, has a preferred particle size distribution of

| | Min | Max |
|---|---|---|
| <3000µm | 70% | 90% |
| >1400µm | 40% | 80% |
| >1000µm | 50% | 80% |
| < 1000µm | 0% | 20% |

The acidic compound of the catalyst of the present disclosure is preferably selected from the group consisting of Al/Si mixed oxides, Al₂O₃, aluminosilicates, silica and zeolites. Al/Si mixed oxides, which are particularly preferred in the present disclosure, refer to a material comprising a mixture of Al₂O₃ and SiO₂, having a neutral structure.

Zeolites as referred to in the present disclosure are understood to be crystalline microporous aluminosilicates which are built up from corner-sharing SiO₄⁻ and AlO₄⁻ tetrahedrons having the general structure Mⁿ⁺_{x/n} [AlO₂]⁻ₓ(SiO₂)_{y}]+ zH₂O with n being the charge of the cation M, typically an alkaline or alkaline earth metal or hydrogen ion, preferably an ion selected from the group consisting of H⁺, Na⁺, Ca²⁺, K⁺ and Mg²⁺, and z defining the number of water molecules incorporated into the crystal structure. Zeolites differ from mixed Al/Si oxides by their defined pore structure and ionic character. In particularly preferred embodiments, the zeolite employed as the acidic compound is selected from the group consisting of Zeolite Y, Zeolite Beta, Zeolite A, Zeolite X, Zeolite L and mixtures thereof, especially Zeolite Y and Zeolite Beta. The listed zeolites are well-known and commercially available. Particularly preferred are zeolites wherein the metal ion M is substituted by a hydrogen. Other specific examples for suitable zeolite-type components to be employed in the present disclosure include but are not limited to ZSM-5, ZSM-11, ZSM-22, ZSM-23, ZSM-35, ZSM-48, ZSM-50, TS-1, TS-2, SSZ-46, MCM-22, MCM-49, FU-9, PSH-3, ITQ-1, EU-1, NU-10, silicalite-1, silicalite-2, boralite-C, boralite-D, BCA, and mixtures thereof.

In particular preferred embodiments, the acidic compound is an Al/Si mixed oxide. The composition of the Al/Si mixed oxide employed as carrier may be adjusted according to need. However, especially favorable results of the depolymerization are achieved in cases where the acidic compound contains Al₂O₃ and SiO₂ in specific amounts. Therefore, in preferred embodiments, the acidic compound contains Al₂O₃ in an amount from 20 to 99 wt.%, preferably from 30 to 80 wt.%, and especially from 40 to 70 wt.%, based on the total weight of the acidic compound. Further, the acidic compound preferably contains SiO₂ in an amount from 1 to 80 wt.%, preferably from 20 to 70 wt.%, and especially from 30 to 60 wt.%, based on the total weight of the acidic compound.

It was surprisingly found that the results of the depolymerization process can even be further improved if the acidic compound contains a slight excess of Al₂O₃. Therefore, in preferred embodiments, the acidic compound comprises an excess of Al₂O₃. Further preferred are embodiments in which the weight ratio of Al₂O₃ to SiO₂ in the acidic compound is from 99:1 to 30:70, preferably from 9:1 to 3:2, and in particular from 4:1 to 3:2.

The determination of the SiO₂ and Al₂O₃ content of the acidic compound can be carried out by atomic emission spectroscopy using an inductively coupled plasma (ICP-AES).

The coating agent employed in the catalyst of the present disclosure is preferably selected from the group consisting of oil, inorganic hydrogel or combinations thereof. As inorganic hydrogel, preference is given to silica hydrogel. With regard to oils employed as the coating agent, preference is given to aromatic-free white mineral oil, preferably based on iso-paraffins. In a preferred embodiment, the oil employed has a kinematic viscosity at 20 °C of 140 to 180 mm²/s, preferably 150 to 170 mm²/s and/or a kinematic viscosity at 40°C of 40 to 80 mm²/s, preferably 50 to 70 mm²/s and/or a kinematic viscosity at 100 °C of 5 to 15 mm²/s, preferably 7 to 10 mm²/s. The kinematic viscosity can be determined according to ISO 3104.

In a further preferred embodiment, the active compound is comprised in the catalyst of the present disclosure in an amount of 0.5 to 6 wt.-%, preferably 2 to 4 wt.-%, based on the total weight of the catalyst.

The catalyst of the present disclosure is preferably obtained by mixing the particulate non-porous support and the coating agent and then adding the acidic compound in the form of a powder to the obtained mixture. The mixture may be optionally heat treated to obtain the catalyst. The heat treatment may, for example, be carried out at a temperature of 100 to 600 °C. In a preferred embodiment, the particulate non-porous support is subjected to a drying step before being mixed with the coating agent. In a preferred embodiment, the method of the present disclosure is carried out in the presence of a catalyst obtained by a process comprising the steps of
a) mixing the particulate non-porous support and the coating agent; and
b) adding the acidic compound in powder form to the mixture of step a).
In an especially preferred embodiment, the catalyst is characterized as follows:
- sand as a particulate non-porous support;
- an Al/Si mixed oxide or a zeolite as acidic compound; and
- mineral oil or silica hydrogel as coating agent.

The preferred catalyst above described can be reactivated by heating thus allowing multiple use and conserving resources. The heat treatment is preferably carried out at a temperature of 500 to 900 °C, especially 600 to 900 °C, alternatively 550-850 °C preferably in oxidative atmosphere such as air or oxygen. The treatment time may be selected by experimental investigation to find the best balance between catalyst activity and energy consumption. In this regard, carbon residue concentration on the catalyst is a parameter to consider. Preferably, carbon residue of regenerated catalyst is less than 20 weight % of the catalyst, preferably less than 15%, more preferably less than 10%, most preferably less than 5%. Without excluding other treatment times, treatment times of 0.5 to 100 hours, preferably 1 to 20 hours, more preferably 2 to 10 hours are possible.

The present disclosure especially regards a method for dealing with waste crosslinked polyethylene. In particular, it was surprisingly found that the method of the present disclosure can be applied to different kinds of crosslinked polyethylene.

In a preferred embodiment, the crosslinked polyethylene in the polymeric waste material is selected from the group consisting of peroxide-crosslinked polyethylene, silane-crosslinked polyethylene, irradiation-crosslinked polyethylene, radically crosslinked polyethylene, azo-crosslinked polyethylene, UV initiated radical crosslinked polyethylene and mixtures thereof.

In some of the major fields of application, in particular pipes and cables, e.g. high-voltage cables, crosslinked polyethylene is used together with multicomponent materials which may also comprise polyolefins. These types of pipes and cables are usually constructed of multiple layers with crosslinked polyethylene being one of them. Further materials are e.g., metals, in particular aluminum, adhesives and other polymers such as EVOH. Although recycling of scrap in the production of pipes and cables is common practice, the focus has so far been on the recovery of Al and limited to the scrap accumulated in the production process. It was surprisingly found that even those multicomponent material containing crosslinked PE can be effectively used as waste material feedstock in the method of the present disclosure. Therefore, in a preferred embodiment, the polymeric waste material is obtained from multicomponent material containing crosslinked PE, in particular high voltage cables containing PEX, PEX pipes, PEX/Al/PEX pipes, PEX/Al/PE-RT pipes and/or PEX/Al/PE pipes. The method of the present disclosure thus directly addresses the need for recycling processes of crosslinked polyethylene pipes.

In an especially preferred embodiment, the waste material comprises or consists of at least one of the following:
i) 5-layer Pipe: PEX / Ad / Alu / Ad / PEX
ii) 5-layer Pipe: PEX/ Ad / Alu / Ad /PE
ii) 5-layer Pipe: PEX/ Ad / Alu / Ad /PERT
iv) 5-layer Pipe: PEX / Ad / EVOH / Ad / PEX
v) 5-layer Pipe: PEX/ Ad / EVOH / Ad /PE
vi) 5-layer Pipe: PEX/ Ad / EVOH / Ad /PERT
vii) 3-layer Pipe: PEX/ Ad / EVOH
viii) 3-layer Pipe: stainless steel/ Ad / PEX
ix) 3-layer Pipe: copper/ Ad / PEX
with PEX depicting the crosslinked polyethylene layer, Ad for adhesives, Alu for aluminum, PE-RT for polyethylene for raised temperature applications, PE standing for polyethylene and EVOH for ethylene vinyl alcohol.

In a further preferred embodiment, the polymeric waste material comprises or consists of a blend comprising shredded PEX pipe waste and plastic waste stream with a melt index (MFI/21.6) of higher than 10 g/10 min with preference given to an amount of plastic waste stream of at least 40 wt.-% of the waste material, preferably 40 to 80 wt.-%, based on the total weight of the waste material.

It constitutes a preferred embodiment of the present disclosure to provide the polymeric waste material in with a bulk density of at least 150 g/cm³. It has been found that the above mentioned value of bulk density greatly helps to achieve a continue flowless depolymerization process and to prevent blockage of feeding lines and reactor fouling. Furthermore, it also helps to obtain low amounts of residues and an enhanced depolymerization reaction increasing the yield of desired products. Preferably, the polymeric waste material has a bulk density of 200 to 700 g/cm³, preferably 200 to 600 g/cm³, more preferably 250 to 550 g/cm³ determined according to DIN 53466.

The polymeric waste material is preferably a shredded pipe with a particle size of <50mm, preferably <30mm, more preferably <20mm and most preferably <15mm.

In a further preferred embodiment , the polymeric waste material is a shredded pipe with a bulk density of at least 300g/cm³ and particle size of <20mm and PE+PP content of at least 90% of organic content.

In order to further enhance the sustainability of cross-linked polyethylene pipes, in particular those which also contain aluminum, the method of the present disclosure employs a waste material feedstock of crosslinked polyethylene further comprising aluminum. In this regard, in a preferred embodiment, the method of the present disclosure further comprises a step of collecting the aluminum from the waste material feedstock.

The method of the present disclosure is characterized by yielding a liquid depolymerization product which can be employed as feedstock for crackers in the production of ethylene. Therefore, preference is given to an embodiment of the present disclosure wherein the gaseous depolymerization product, which is subsequently condensed to the desired liquid depolymerization, is released from the screw reactor by gas release units which are placed along the screw conveyer. This way, different vapor fractions can be sequentially released along the screw and can be collected individually.

It was surprisingly found that the method of the present disclosure produces a highly selective depolymerization product. The selectivity is still improved by the specific combination of screw reactor and particulate catalyst used in the present disclosure. A still further improvement in selectivity can be obtained by hot gas filtration which can be favorably combined with collecting the gaseous depolymerization product. Therefore, in a preferred embodiment, collection of the gaseous depolymerization product comprises a step of hot gas filtration.

The gaseous depolymerization product obtained in the process of the present disclosure is at least partially condensed to yield a liquid depolymerization product which may be further processed as cracker feedstock. In a preferred embodiment of the method of the present disclosure, condensing of the gaseous depolymerization product is achieved by sequentially passing the gaseous depolymerization product through a plurality of condensing units. The condensing units may be operated at different temperatures to help separate the high boiling fraction, medium boiling fraction and high boiling fraction of the depolymerization product.

Apart from showing a high selectivity, it was surprisingly found that the method of the present disclosure also allows for a comparatively short residence time of the waste material in the reactor. Therefore, preference is given to an embodiment of the present disclosure wherein the residence time of the waste material is no more than 60 minutes, preferably no more than 45 minutes.

It was further surprisingly found that the method of the present disclosure can be conducted at favorable low temperatures which, in combination with the short residence time of the waste material in the reactor, allows to preserve valuable energy and reduce the carbon footprint. In a preferred embodiment, the reactor is thus operated at a temperature of 350 to 650 °C, preferably 400 to 590 °C, more preferably from 430 to 500°C. In a further preferred embodiment, the reactor is operated at a pressure of 0.7 to 10 bar_{g}.

In a preferred embodiment, the reactor is heated electrically. This way of heating, in particular in combination with the sequential release of the different vapor fractions along the screw conveyor, is believed to further support the detailed mass and heat balancing of the depolymerization process which allows for the high selectivity of the method of the present disclosure.

The depolymerization product obtained in the method of the present disclosure may contain non-liquifiable fractions. In a preferred embodiment, those non-liquifiable fractions are re-introduced into the reactor to minimize production of non-usable depolymerization waste. Also, it was found that the catalyst employed in the method can be employed in more than one cycle and can be re-activated by loss of activity. Therefore, in a preferred embodiment, at least part of the catalyst is re-introduced into the reactor.

Within the course of the present disclosure, it was found that the content of methane in the gaseous depolymerization can serve as an indicator of the progress of depolymerization. Therefore, in a preferred embodiment, the content of methane in the collected gaseous depolymerization product is monitored.

The method of the present disclosure yields a depolymerization product of particular selectivity. In a preferred embodiment, the liquid depolymerization product obtained comprises from 35 to 45 wt.-% of a high boiling fraction, from 40 to 50 wt.-% of a medium boiling fraction and from 15 to 25 wt.-% of a low boiling fraction.

The process of the present disclosure surprisingly generates little to no char. Therefore, in preferred embodiments, the residue of the depolymerization process of the present disclosure has a char content of less than 5 wt.%, preferably less than 2 wt.%, based on the total weight of the product.

The obtained liquid depolymerization product may be further separated. Therefore, the process of the present disclosure further comprises a step of distilling the liquid depolymerization product.

It was also found that the process of the present disclosure yields a depolymerization product with a high gaseous content. In a preferred embodiment, the gaseous content in the depolymerization product after step iv) is preferably more than 30 wt.-%, more preferably more than 50 wt.-% and in particular more than 60 wt.-% and especially more than 70 wt.-%, of gaseous components, based on the weight of polymeric fraction of the polymeric waste material, said gaseous components preferably comprising more than 50 wt.-% of olefins, based on the total weight of the gaseous components.

The gaseous fraction of the depolymerization product is further distinguished by high content of monomeric olefinic C₂-C₄-compounds which are especially useful for further processing, e.g. for the production of polymers. Due to the high amount of said compounds generated during depolymerization, the gaseous depolymerization product may be also directly used as feedstock in cracking processes and subsequent polymerization. The gaseous product comprising light olefins and light alkanes can, for example be transferred to a downstream cracker by passing the ovens to produce polymerization grade monomer streams. Other side products such as ethane, propane and butanes will be cracked in the oven. The usually required steps of treating the product obtained after depolymerization to obtain the desired monomers can thus be bypassed, saving valuable energy and reducing CO₂ output.

### i) Liquid depolymerization product

The obtained liquid depolymerization product preferably has a low content of aromatic compounds and in particular a low content of polycyclic aromatic compounds and asphaltanes. The liquid depolymerization product obtained by the process of the present disclosure is accordingly characterized by a low content of aromatic and olefinic components as well as a high degree of purity.

Preferably, the content of aromatic compounds in the obtained liquid depolymerization product is less than 10 mol%, preferably less than 5 mol%, and in particular no more than 3 mol%, the content of aromatic components being measured as contents of aromatic protons in mol% as determined by ¹H-NMR -spectroscopy.

Further, the liquid depolymerization product obtained by the depolymerization process of the present disclosure is characterized by a low content of olefinic compounds. The content of olefinic compounds in the liquid depolymerization product is preferably less than 5 mol%, more preferably less than 3 mol%, even more preferably less than 1.5 mol%, and in particular no more than 1 mol%, based on the total number of hydrocarbon protons, the content of olefinic compounds determined based on the contents of olefinic protons as determined by ¹H-NMR -spectroscopy.

Another measure for the content of double bonds in a given sample is the Bromine number (BrNo.) which indicates the degree of unsaturation. In preferred embodiments, the liquid depolymerization product obtained by the process of the present disclosure has a Bromine number, expressed as gram bromine per 100 grams of sample, of less than 25, preferably from 0.1 to 20, more preferably from 0.2 to 15, even more preferably from 0.3 to 10 and in particular from 0.5 to 5, determined according to ASTM D1159-01.

The liquid depolymerization product obtained in the process of the present disclosure has preferably a boiling range from 30 to 650 °C, more preferably from 50 to 250 °C. By separation techniques such as distillation, the depolymerization product may be separated into hydrocarbon fractionations of different boiling ranges, for example a light naphtha fraction mainly containing C₅ and C₆ hydrocarbons having a boiling range from 30 °C and 130 °C, a heavy naphtha fraction mainly containing C₆ to C₁₂ hydrocarbons having a boiling range from 130 °C to 220 °C, a kerosene fraction mainly containing C₉ to C₁₇ hydrocarbons having a boiling range from 220 °C to 270 °C or into other high boiling point fractions such as diesel fuel, fuel oil or hydrowax.

It was further surprisingly found that the liquid depolymerization product contains little to no solid residue which is usually found in common depolymerization processes. In preferred embodiments, the content of residues of the liquid depolymerization product upon evaporation, determined according to ASTM D381, is no more than 5 ppm (w).

### ii) Gaseous depolymerization product

The gaseous depolymerization product obtained preferably has a low content of low molecular hydrocarbons such as methane or ethane. As mentioned above, the gaseous depolymerization product preferably contains high amounts of higher olefins such as ethylene propylene and butenes which are commonly desired for polyolefin production. Accordingly, the gaseous depolymerization product obtained by the process of the present disclosure is characterized by a high content of any of ethylene, propylene and butenes and/or a low content of saturated low molecular hydrocarbons, in particular hydrocarbons of the general formula CₙH₂ₙ₊₂ wherein n is a real number ranging from 1 to 4.

In a preferred embodiment, the gaseous depolymerization product of the process of the present disclosure is therefore characterized by a content of methane of at most 5 wt.-%, preferably at most 4 wt.-%, more preferably at most 3 wt.-%, most preferably at most 2 wt.-%, especially at most 0,5-1,5 wt.-%, based on the total weight of the gaseous depolymerization product after step iv) of the method of the present disclosure.

It was surprisingly found that the gaseous depolymerization product obtained in the method of the present disclosure contained a high amount of low molecular olefinic compounds, especially of the CₙH₂ₙ variety with n = 2-4. The gaseous fraction could thus be directly used as feedstock for further processing in a cracker downstream, e.g. a raw gas compressor to obtain purified monomer streams, and thereafter for the subsequent production of polymers, allowing bypassing the highly energy consuming stream cracking ovens usually required while at the same time reducing the output of CO₂. In a preferred embodiment, the gaseous depolymerization product of the method of the present disclosure is therefore characterized by a content of compounds of the general formula CₙH₂ₙ (olefins) with n = 2-4 of at least 50 wt.-%, preferably at least 60 wt.-%, more preferably at least 65 wt.-%, most preferably at least 70 wt.-%, especially at least 75 wt.-%, based on the total weight of the gaseous depolymerization product after step iv) of the method of the present disclosure.

The gaseous depolymerization product may contain small quantities of HCl, HCN, H₂S, H₂O, NH₃, COS etc. which can be optionally separated in a refining step before the introduction to the steam cracker downstream segments.

The method of the present disclosure will be explained in more detail with reference to the following examples which are by no means to be understood as limiting the scope and spirit of the disclosure.

The following analytical methods were employed:
1) GC MS was used for liquid and gas analysis.
2) Char residue was determined according to mass balance after decoking the residues of the reactor at 800 °C.
3) Liquid contents were characterized using simulated distillation (SimDist) analysis according to ASTM D 7213 : 2012.
4) The total content of unsaturated components in the liquid condensates were characterized via Bromine number determination using a 848 Titrino Plus (Metrohm AG, Herisau, Switzerland) equipped with an double PT-wire electrode which has integrated a PT1000 temperature sensor, and a 10 ml buret in accordance with ASTM D1159-01 as described in Metrohm Application Bulletin 177/5e, December 2018. The Bromine number (BrNo.) represents the amount of bromine in grams absorbed by 100 grams of a sample.
5) ¹H-NMR analysis was conducted by dissolving a sample of the liquid condensate in CDCl₃ and characterizing the sample using proton NMR spectroscopy. Aromatic, olefinic and aliphatic protons were assigned according to the chemical shifts summarized in Table 1:

**Table 1 - Integral Regions in ¹H-NMR spectroscopy**

| Peak Assignment | ¹H Chemical Shift (ppm) |
|---|---|
| I₁ (Aromatic Protons) | 8.25 - 7.27 |
| CDCl₃ -Solvent | 7.26 |
| I₂ (Aromatic Protons) | 7.25 - 6.60 |
| I₃ (Olefinic Protons - Type 2 ) | 6.60 - 5.95 |
| I₄ (Olefinic Protons - Type 1 ) | 5.95 - 5.67 |
| I₅ (Olefinic Protons - Type 2 ) | 5.67 - 5.35 |
| I₆ (Olefinic Protons - Type 3 ) | 5.35 - 5.15 |
| I₇ (Olefinic Protons - Type 1 ) | 5.15 - 4.85 |
| I₈ (Olefinic Protons - Type 4 ) | 4.85 - 4.40 |
| I₉... (Paraffinic Protons) | 4.40 - 0.25 |

The listed types of olefinic protons are assumed to correspond to the following structures:

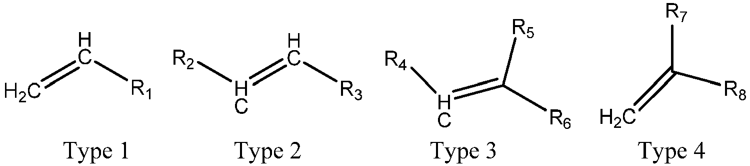

The amounts of aromatic, olefinic and aliphatic protons may be determined based on the assigned peak integrals according to the following equations: $Mol % Aromatic Protons = \left[\left({I}_{1}+{I}_{2}\right)/\left({I}_{1}+{I}_{2}+{I}_{3}+{I}_{4}+{I}_{5}+{I}_{6}+{I}_{7}+{I}_{8}+{I}_{9}\right)\right] %$ $Mol % Olefinic Protons Type 1 = \left[\left({I}_{4}+{I}_{7}\right)/\left({I}_{1}+{I}_{2}+{I}_{3}+{I}_{4}+{I}_{5}+{I}_{6}+{I}_{7}+{I}_{8}+{I}_{9}\right)\right] %$ $Mol % Olefinic Protons Type 2 = \left[\left({I}_{3}+{I}_{5}\right)/\left({I}_{1}+{I}_{2}+{I}_{3}+{I}_{4}+{I}_{5}+{I}_{6}+{I}_{7}+{I}_{8}+{I}_{9}\right)\right] %$ $Mol % Olefinic Protons Type 3 = \left[\left({I}_{6}\right)/\left({I}_{1}+{I}_{2}+{I}_{3}+{I}_{4}+{I}_{5}+{I}_{6}+{I}_{7}+{I}_{8}+{I}_{9}\right)\right] %$ $Mol % Olefinic Protons Type 4 = \left[\left({I}_{8}\right)/\left({I}_{1}+{I}_{2}+{I}_{3}+{I}_{4}+{I}_{5}+{I}_{6}+{I}_{7}+{I}_{8}+{I}_{9}\right)\right] %$ $Mol % Paraffinic Protons = \left[\left({I}_{9}\right)/\left({I}_{1}+{I}_{2}+{I}_{3}+{I}_{4}+{I}_{5}+{I}_{6}+{I}_{7}+{I}_{8}+{I}_{9}\right)\right] %$
6) The water content of the catalyst was determined using a Sartorius MA45 (Sartorius AG, Goettingen, Germany) on a sample of 0.5 to 1 g at 180°C.
7) For the determination of a pH value of the hydrodepolymerization products, by extraction of a liquid sample of the hydrodepolymerization product was extracted with water in a volume ratio water:sample of 1:5 and the pH value of the aqueous solution was measured.
8) Particle size distribution of the particulate non-porous support and the catalyst were determined according to Coulter counter analysis in accordance with ASTM D4438.
9) Properties of the employed organic waste material feedstock were determined as follows:

As the composition of the waste material may vary, samples from 20 to 100 g of the waste material were milled and analyzed. Alternatively, a pelletized sample of the polymeric waste was analyzed.

The following methods are used:
i) Total Volatiles (TV) were measured as the weight loss of a 10 g sample at 100 °C and after 2 hours at 200 mbar.
ii) Water content was determined by Karl-Fischer titration using an apparatus from Metrohm 915 KF Ti-Touch equipped with a PT100 indicator electrode for volumetric KF titration according to Metrohm Application Bulletin 77/3e in compliance with ASTM E203.
iii) IR-Spectroscopy was used for a qualitative identification of various polymers (PP, PE, PS, PA, PET, PU, Polyester) and additives such as CaCO₃
iv) Standard elemental analysis was used for determination of wt.% of H, C, N (DIN 51732: 2014-07) and S (tube furnace, ELTRA GmbH, Haan, Germany, DIN 51724-3: 2012-07).
v) ¹H-NMR was used for determining the composition of polymers soluble in solvents adequate for recording a ¹H-NMR spectrum: PE/PP balance (copolymers are also included), PET, PS
vi) Ash Content analysis of plastics was determined at 800 °C according to DIN EN ISO 3451-1 (2019-05).
vii) Bulk density of the polymer waste was determined according to DIN 53466.
viii) Corrosivity was determined as the pH value of an aqueous solution after a contact time of 3 h (5 g sample in 50 ml distilled water)
ix) Inductively coupled plasma atomic emission spectroscopy (ICP-AES) was used for quantitative element determination (total chlorine content, content of Si or metals)

Various catalysts were prepared and tested in depolymerizations of different polymeric waste materials.

As the particulate, non-porous support of the catalyst, sand having a particle size distribution as summarized in Table 2 was employed.

**Table 2: Particle size distribution**

| | Sand wt% |
|---|---|
| >1400µm | 40,55% |
| > 1400µm- >1000µm | 55,30% |
| < 1000µm | 4,15% |

with 99% of the particles being smaller than 3 mm. The sand employed was pre-dried at 80 °C for 24 h in a drying oven with circulating air.

### Catalyst #4:

The acidic compound was mixed with 25 kg of sand to obtain a catalyst as shown in Table 4.

### Catalysts #3:

25.0 kg of sand were placed into a 60 L steel barrel with screw cap and equipped with a Teflon inlay. 350 ml mineral oil were added (corresponding to 1.4 wt% with respect to sand). The drum was placed on a Drum Hoop Mixer and rotated for 1 hour (about 100 rpm). 24.5 kg of the obtained mixture was placed in another drum and 500 g of the corresponding acidic component (corresponding to a 2 wt% loading) were added. The drum was placed on a drum hoop mixer and rotated for 1 hour (about 100 rpm). At the end of the mixing process a free-flowing catalyst was obtained with an even distribution of the particles of the acidic component on the surface of the sand particles.
As the mineral oil Ondina X 432 was used which is commercially available from Shell having the following properties:

**Table 3:**

| Property | Method | Shell Ondina X 432 |
|---|---|---|
| Kinematic Viscosity at 20°C in mm²/s | ISO 3104 | 165 |
| Kinematic Viscosity at 40°C in mm²/s | ISO 3104 | 59 |
| Kinematic Viscosity at 100°C in mm²/s | ISO 3104 | 9.0 |

### Preparation of Catalysts #1-#2

The catalyst of the present disclosure using silica hydrogel as coating agent was prepared as follows:
25.0 kg of sand were placed into a 60 L steel barrel with screw cap and equipped with a Teflon inlay. 500 ml water were added (corresponding to 2,0 wt% with respect to sand) and the drum was placed on a Drum Hoop Mixer and rotated for 1 hour (about 100 rpm). 24.5 kg of the obtained mixture was placed in another drum and 1000 g of a 1:1 milled free-flowing mixture of silica hydrogel and the acidic compound (corresponding to a 2 wt% loading) and were added. The drum was placed on a drum hoop mixer and rotated for 1 hour (about 100 rpm). At the end of the mixing process a free-flowing catalyst was obtained with an even distribution of the particles of the acidic compound on the surface of the sand particles. The obtained mixture was dried at 120 °C vacuum for 6 h.

The silica hydrogel was prepared according to EP1290042, example 1. The solid content of the hydrogel sample was 20 wt%. The D50 of the milled mixture of silica hydrogel and acidic components were between 80-100 µm, in accordance with ASTM D4438.
Table 4 summarizes the catalysts employed with the amount of the acidic compound given in wt.-% with respect to sand.

**Table 4**

| Catalyst # | coating agent | acidic compound | amount acidic compound |
|---|---|---|---|
| 1 | silica hydrogel | Zeolyst Beta | 2 |
| 2 | silica hydrogel | Zeolyst ZSM-5 | 2 |
| 3 | oil | Zeolyst Beta | 2 |
| 4 | none | Zeolyst Beta | 2 |

### Acidic compounds:

Zeolyst ZSM-5 and Zeolyst Beta (CP811E-75) commercially available from PQ Corporation, Malvern, PA, USA

### Feedstock:

The following organic waste materials were employed as feedstocks:
A) Shredded Multilayer PEX pipe waste with an Aluminum layer (Particle size <20mm)
B) Shredded PEX pipe waste with EVOH (Particle size <20mm)
C) Shredded PEX pipe waste with an Aluminum layer (Particle size <20mm)
D) Shredded monolayer PEX pipe waste (Particle size <20mm)
E) Shredded high voltage cable crosslinked waste (Particle size <20mm)
F) shredded and pelletized mixed plastic waste from household packaging (comparison)
G) 1:1 mixture of feedstock D) and a post-consumer recyclate with an MFR (21,6) of 20 g/10 min blended at 190°C to obtain extrudable pellets of MFR (21,6) of 15g/10 min.

The properties of the feedstocks averaged on analysis of three samples are summarized in Table 5.

**Table 5:**

| | | | | | | Polymer composition analysis | | | | | |
|---|---|---|---|---|---|---|---|---|---|---|---|
| Feed-stock | Ash | TV | BD | Cl | PE+PP | PE | PP | PET | PS | PA | Other cont. |
| | wt% | wt% | g/cm³ | wt% | wt% | wt% | wt% | wt% | wt% | wt% | wt% |
| A | 25% | 0.5 | 356 | <0.01 | 75 | ~75 | - | - | - | - | 25 |
| B | 0.5 | 1 | 316 | <0.01 | | >95 | - | - | - | - | 2 |
| C | 11.2 | 1.5 | 326 | <0.01 | | >87 | - | - | - | - | 13 |
| D | <0.1 | 1.7 | 360 | <0.01 | | >98 | - | - | - | - | - |
| E | 6.5 | 0.6 | 480 | <0.01 | | >93 | - | - | - | - | 7 |
| F | 4.8 | 4 | 230 | 0.2 | 88 | 72 | 16 | 2.3 | 1.2 | - | 8.5 |
| G | <0.1 | 0.3 | 529 | <0.01 | | >98 | - | - | - | - | - |

| | | | | | | | | | | | |
|---|---|---|---|---|---|---|---|---|---|---|---|
| Ash: ash content TV: total volatiles BD: bulk density Cl: total chlorine content PE : polyethylene content PP : polypropylene content PET: content of polyethylene terephthalate PS: polystyrene content PA: polyamide content Other cont.: content of other contaminants | | | | | | | | | | | |

The feedstock and catalyst were introduced into a reactor with a screw conveyor at 450 °C and 30 min residence time. The product compositions of the depolymerization process are summarized in Table 6. The obtained gaseous fractions were further separated into liquid and gaseous depolymerization products by condensation. The amounts of the obtained fractions are also given in Table 6.

**Table 6: Process parameter and mass balance**

| Run# | Cat# | Feedstock | %Wax¹ | %Liquid²) | %H₂O | %Gas | % Residue | %loss |
|---|---|---|---|---|---|---|---|---|
| 1 | 1 | A | 0 | 11.7 | 0.7 | 60.3 | 28.4 | -1.1 |
| 2 | 1 | B | 0 | 20.3 | 0.9 | 82 | 0 | -3.2 |
| 3 | 1 | C | 0 | 13.2 | 0.6 | 77.4 | 12.1 | -3.3 |
| 4 | 1 | D | 0 | 18.5 | 0.8 | 81.7 | 1.9 | -2.9 |
| 5 | 1 | E | 0 | 16.6 | 0.9 | 74.4 | 7.2 | 0.9 |
| C6 | 1 | F | 0 | 36.2 | - | 52.9 | 8.9 | 1.9 |
| C7 | 4 | F | (0) | 46.7*⁾ | - | 32.4 | 10.1 | 10.7 |
| C8 | no Cat. | F | (0) | 52.4*⁾ | - | 29 | 18 | 1 |

| | | | | | | | | |
|---|---|---|---|---|---|---|---|---|
| 1) solid at room temperature; 2) liquid at room temperature *): visible yellowish particles after cooling, liquid fraction turned to a suspension containing waxes | | | | | | | | |

Runs #1-5 demonstrate a very effective depolymerization of crosslinked plastic waste with over 70% of gas generation with hydrocarbon content of more than 95%. The obtained HC liquids show low viscosity and showed a brown/red color. In contrast, the obtained HC liquids of runs #C7-C8 were black colored with high viscosity and visible wax particles. Remarkable is also the low amount of methane, CO and CO₂ obtained in inventive runs #1-5 which demonstrates the high selectivity of the employed catalyst towards olefins.

Run #4 was repeated with Feedstock G. As expected very similar results to feedstock D were obtained.

In Run #C7, a high amount of loss due reactor fouling was observed.

The resulting residue/sand mixture from Runs #1 and #3 was sieved over a 2 mm grid. The pieces of aluminum layer were separated effectively from the sand/residue mixture enabling further recycling of aluminum content of the PEX pipe of up to 90% of the total Al content even by simple sieving.

In particular, in Run #1 18% Al and in Run #3 9% Al yield, based on starting Feedstock A respectively, could be recovered.

Comparative runs with the polymeric waste material with bulk density of 60 respectively 150g/cm³ were also carried out in the same reactor set-up. The runs were impacted repeatedly by blockage of feeding line and reactor fouling which rendered the runs troublesome.
Table 7 shows the mass balance of the gaseous depolymerization product obtained in Runs #1-8.

**Table 7: mass balance of the gaseous depolymerization product**

| | | | | | | | | |
|---|---|---|---|---|---|---|---|---|
| Run | 1 | 2 | 3 | 4 | 5 | C6 | C7 | C8 |
| Feedstock | A | B | C | D | E | F | F | F |
| Catalyst # | 1 | 1 | 1 | 1 | 1 | 1 | 4 | no Cat. |

| Components | wt.-% | wt.-% | wt.-% | wt.-% | wt.-% | wt.-% | wt.-% | wt.-% |
|---|---|---|---|---|---|---|---|---|
| H₂ | 0 | 0.1 | 0.1 | 0.1 | 0.1 | 0.0 | 0.0 | 0.0 |
| CO | 0.6 | 0 | 0 | 0.0 | 0.0 | 11.4 | 11.3 | 6.4 |
| CO₂ | 3.7 | 0.6 | 0.5 | 0.5 | 2.0 | 22.6 | 22.3 | 36.5 |
| CH₄ | 0.5 | 0 | 0 | 0.0 | 0.6 | 5.5 | 5.4 | 3.6 |
| C₂H₆ | 0.4 | 0.6 | 0.7 | 0.5 | 0.4 | 7.6 | 7.5 | 5.8 |
| C₂H₄ | 1.1 | 1.4 | 1.5 | 2.0 | 1.6 | 6.8 | 6.7 | 5.1 |
| C₃H₈ | 3.4 | 2.7 | 3.2 | 3.4 | 0.7 | 7.1 | 7.0 | 4.4 |
| C₃H₆ | 34.6 | 28.8 | 28.3 | 29.6 | 32.1 | 15.1 | 14.9 | 15.6 |
| Butanes | 27.9 | 23.4 | 22.6 | 23.2 | 22.5 | 3.6 | 3.5 | 1.8 |
| Butenes | 27.7 | 42.4 | 43.2 | 40.7 | 40.0 | 8.9 | 8.9 | 10.8 |
| Rest | 0 | 0 | 0 | 0.0 | 0.0 | 11.4 | 12.5 | 10.2 |
| Olefins | 63.4 | 72.6 | 73.0 | 72.3 | 73.7 | 30.8 | 30.5 | 31.4 |
| total HC | 95.6 | 99.3 | 99.5 | 99.4 | 97.9 | 54.6 | 54.0 | 46.9 |
| Olefins/HC | 66.3 | 73.1 | 73.4 | 72.7 | 75.3 | 56.3 | 56.4 | 66.9 |

## Claims

1. Method for the production of a cracker feedstock by depolymerization of a polymeric waste material comprising crosslinked polyethylene (PEX), the method comprising the steps of
i) introducing the waste material feedstock into a pyrolysis reactor, preferably a reactor with a screw conveyer;
ii) mixing the waste material with a depolymerization catalyst comprising an acidic compound;
iii) depolymerizing the waste material in an oxygen-free atmosphere at a temperature ranging from 400 to 590°C to obtain a gaseous depolymerization product;
iv) collecting and condensing at least part of the gaseous depolymerization product to obtain the desired cracker feedstock.

2. The method of claim 1, **characterized in that** the acidic compound of the catalyst is deposited on a particulate non-porous support with the aid of a coating agent.

3. The method of claim 2 **characterized in that** the acidic compound is selected from the group consisting of Al/Si mixed oxides, Al₂O₃, aluminosilicates, silica and zeolites, in particular from the group of Zeolite Y, Zeolite Beta, Zeolite A, Zeolite X, Zeolite L and mixtures thereof, especially from the group consisting of Zeolite Y and Zeolite Beta.

4. The method of claim 2 **characterized in that** the particulate non-porous support is selected from the group consisting of sand, glass beads and metal particles and the coating agent is selected from the group consisting of oil, inorganic hydrogel and combinations thereof.

5. The method of any of the forgoing claims, **characterized in that** the waste material further comprises aluminum and **in that** the method further comprises a step of collecting the aluminum from the waste material.

6. The method of any of the forgoing claims, **characterized in that** the polymeric waste material contains in the in the polymeric fraction an amount of non-polyolefin components lower than 12 wt.%, and preferably less than 10 wt.% based on the total weight of the dry weight polymeric fraction of the waste material feedstock.

7. The method of any of the forgoing claims, **characterized in that** the polymeric waste material in with a bulk density of at least 150 g/cm³, preferably 200 to 700 g/cm³, preferably 200 to 600 g/cm³, more preferably 250 to 550 g/cm³ determined according to DIN 53466.

8. The method of any of the forgoing claims, **characterized in that** the crosslinked polyethylene in the polymeric waste material is selected from the group consisting of peroxide-crosslinked polyethylene, silane-crosslinked polyethylene, irradiation-crosslinked polyethylene, radically crosslinked polyethylene, azo-crosslinked polyethylene, UV initiated radically crosslinked polyethylene and mixtures thereof.

9. The method of any of the forgoing claims, **characterized in** the polymeric waste material is obtained from PEX pipes, PEX/Al/PEX pipes, PEX/A1/PE-RT pipes and/or PEX/Al/PE pipes.

10. The method of any of the forgoing claims, **characterized in that** the polymeric waste material comprises or consists of shredded PEX pipe waste and plastic waste stream with a melt index (MFI/21.6) of higher than 10 g/10 min with preference given to an amount of plastic waste stream of at least 40 wt.-% of the waste material, preferably 40 to 80 wt.-%, based on the total weight of the waste material.

11. The method of any of the forgoing claims, **characterized in that** it is carried out in a reactor with a screw conveyer and **in that** the gaseous depolymerization product is released from the reactor by gas release units placed along a screw conveyer.

12. The method of any of the forgoing claims, **characterized in that** the residence time of the waste material in the reactor is no more than 60 minutes, preferably no more than 45 minutes.

13. The method of any of the forgoing claims, **characterized in that** the reactor is operated at a pressure of 0.7 to 10 bar_{g}.

14. The method of any of the forgoing claims, **characterized in that** the collected depolymerization product comprises more than 30 wt.-% more preferably more than 50 wt.-% and in particular more than 60 wt.-%, of gaseous components, based on the weight of polymeric fraction of the polymeric waste material, with the said gaseous components preferably comprising more than 50 wt.-% of olefins, based on the total weight of the gaseous components.

15. The method of any of the forgoing claims, **characterized in that** the depolymerization liquid product comprises from 35 to 45 wt.-% of a high boiling fraction, from 40 to 50 wt.-% of a medium boiling fraction, and from 15 to 25 wt.-% of a low boiling fraction.

## Patentansprüche

1. Verfahren zur Herstellung eines Cracker-Einsatzmaterials durch Depolymerisation eines polymeren Abfallmaterials, das vernetztes Polyethylen (PEX) umfasst, wobei das Verfahren die folgenden Schritte umfasst:
i) Einbringen des Abfallmaterials als Ausgangsmaterial in einen Pyrolysereaktor, vorzugsweise einen Reaktor mit einer Förderschnecke;
ii) das Abfallsubstrat mit einem Depolymerisationskatalysator, der eine saure Verbindung enthält, vermischen;
iii) Depolymerisierung des Abfallmaterials in einer sauerstofffreien Atmosphäre bei einer Temperatur im Bereich von 400 bis 590 °C, um ein gasförmiges Depolymerisationsprodukt zu erhalten;
iv) Auffangen und Kondensieren zumindest eines Teils des gasförmigen Depolymerisationsprodukts, um das gewünschte Cracker-Einsatzmaterial zu erhalten.

2. Verfahren nach Anspruch 1, **dadurch gekennzeichnet, dass** die saure Verbindung des Katalysators mit Hilfe eines Beschichtungsmittels auf einem teilchenförmigen, nicht porösen Träger abgeschieden wird.

3. Verfahren nach Anspruch 2, **dadurch gekennzeichnet, dass** die saure Verbindung aus der Gruppe ausgewählt ist, die aus Al/Si-Mischoxiden, _{Al2O3}, Alumosilikaten, Siliciumdioxid und Zeolithen besteht, insbesondere aus der Gruppe von Zeolith Y, Zeolith Beta, Zeolith A, Zeolith X, Zeolith L und Mischungen davon, vor allem aus der Gruppe, die aus Zeolith Y und Zeolith Beta besteht.

4. Verfahren nach Anspruch 2, **dadurch gekennzeichnet, dass** der partikuläre, nichtporöse Träger aus der Gruppe ausgewählt ist, die aus Sand, Glasperlen und Metallpartikeln besteht, und das Beschichtungsmittel aus der Gruppe ausgewählt ist, die aus Öl, anorganischem Hydrogel und Kombinationen davon besteht.

5. Verfahren nach einem der vorstehenden Ansprüche, **dadurch gekennzeichnet, dass** das Abfallmaterial ferner Aluminium umfasst und dass das Verfahren ferner einen Schritt zum Gewinnen des Aluminiums aus dem Abfallmaterial umfasst.

6. Verfahren nach einem der vorstehenden Ansprüche, **dadurch gekennzeichnet, dass** das polymere Abfallmaterial in der polymeren Fraktion einen Anteil an Nicht-Polyolefin-Komponenten von weniger als 12 Gew.-% und vorzugsweise weniger als 10 Gew.-%, bezogen auf das Gesamtgewicht der trockenen polymeren Fraktion des Abfallmaterials als Ausgangsmaterial, enthält.

7. Verfahren nach einem der vorstehenden Ansprüche, **dadurch gekennzeichnet, dass** das polymere Abfallmaterial eine Schüttdichte von mindestens 150 g/cm³, vorzugsweise 200 bis 700 g/cm³, noch bevorzugter 200 bis 600 g/cm³ und am meisten bevorzugt 250 bis 550 g/cm³ aufweist, bestimmt gemäß DIN 53466.

8. Verfahren nach einem der vorstehenden Ansprüche, **dadurch gekennzeichnet, dass** das vernetzte Polyethylen im polymeren Abfallmaterial aus der Gruppe ausgewählt ist, die aus peroxidvernetztem Polyethylen, silanvernetztem Polyethylen, strahlungsvernetztem Polyethylen, radikalvernetztem Polyethylen, azovernetztem Polyethylen, UV-initiiertem radikalvernetztem Polyethylen und Mischungen davon besteht.

9. Verfahren nach einem der vorstehenden Ansprüche, **dadurch gekennzeichnet, dass** das polymere Abfallmaterial aus PEX-Rohren, PEX/Al/PEX-Rohren, PEX/Al/PE-RT-Rohren und/oder PEX/Al/PE-Rohren gewonnen wird.

10. Verfahren nach einem der vorstehenden Ansprüche, **dadurch gekennzeichnet, dass** das polymere Abfallmaterial zerkleinerte PEX-Rohrabfälle und einen Kunststoffabfallstrom mit einem Schmelzindex (MFI/21,6) von mehr als 10 g/10 min umfasst oder daraus besteht, wobei vorzugsweise der Anteil des Kunststoffabfallstroms mindestens 40 Gew.-% des Abfallmaterials beträgt, vorzugsweise 40 bis 80 Gew.-%, bezogen auf das Gesamtgewicht des Abfallmaterials.

11. Verfahren nach einem der vorstehenden Ansprüche, **dadurch gekennzeichnet, dass** es in einem Reaktor mit einer Förderschnecke durchgeführt wird und dass das gasförmige Depolymerisationsprodukt durch entlang einer Förderschnecke angeordnete Gasabgabeeinheiten aus dem Reaktor abgegeben wird.

12. Verfahren nach einem der vorstehenden Ansprüche, **dadurch gekennzeichnet, dass** die Verweilzeit des Abfallmaterials im Reaktor nicht mehr als 60 Minuten, vorzugsweise nicht mehr als 45 Minuten beträgt.

13. Verfahren nach einem der vorstehenden Ansprüche, **dadurch gekennzeichnet, dass** der Reaktor bei einem Druck von 0,7 bis 10 barg betrieben wird.

14. Verfahren nach einem der vorstehenden Ansprüche, **dadurch gekennzeichnet, dass** das gesammelte Depolymerisationsprodukt mehr als 30 Gew.-%, vorzugsweise mehr als 50 Gew.-% und insbesondere mehr als 60 Gew.-%, an gasförmigen Bestandteilen, bezogen auf das Gewicht der polymeren Fraktion des polymeren Abfallmaterials, umfasst, wobei die genannten gasförmigen Bestandteile vorzugsweise mehr als 50 Gew.-% Olefine, bezogen auf das Gesamtgewicht der gasförmigen Bestandteile, umfassen.

15. Verfahren nach einem der vorstehenden Ansprüche, **dadurch gekennzeichnet, dass** das flüssige Depolymerisationsprodukt 35 bis 45 Gew.-% einer hochsiedenden Fraktion, 40 bis 50 Gew.-% einer mittelsiedenden Fraktion und 15 bis 25 Gew.-% einer niedrigsiedenden Fraktion umfasst.

## Revendications

1. Procédé de production d'une charge d'alimentation pour craqueur par dépolymérisation d'un déchet polymère comprenant du polyéthylène réticulé (PEX), ledit procédé comprenant les étapes suivantes :
i) introduire la matière première constituée de déchets dans un réacteur de pyrolyse, de préférence un réacteur équipé d'un convoyeur à vis ;
ii) le mélange des déchets avec un catalyseur de dépolymérisation comprenant un composé acide ;
iii) dépolymériser les déchets dans une atmosphère exempte d'oxygène à une température comprise entre 400 et 590 °C afin d'obtenir un produit de dépolymérisation gazeux ;
iv) récupérer et condenser au moins une partie du produit de dépolymérisation gazeux afin d'obtenir la charge d'alimentation souhaitée pour le craqueur.

2. Procédé selon la revendication 1, **caractérisé en ce que** le composé acide du catalyseur est déposé sur un support particulaire non poreux à l'aide d'un agent d'enrobage.

3. Procédé selon la revendication 2, **caractérisé en ce que** le composé acide est choisi parmi le groupe constitué des oxydes mixtes Al/Si, de l'Al₂O₃, des aluminosilicates, de la silice et des zéolithes, en particulier parmi le groupe comprenant la zéolite Y, la zéolite bêta, la zéolite A, la zéolite X, la zéolite L et leurs mélanges, notamment parmi le groupe constitué de la zéolite Y et de la zéolite bêta.

4. Procédé selon la revendication 2, **caractérisé en ce que** le support particulaire non poreux est choisi parmi le groupe constitué du sable, des billes de verre et des particules métalliques, et l'agent d'enrobage est choisi parmi le groupe constitué de l'huile, d'un hydrogel inorganique et de leurs combinaisons.

5. Procédé selon l'une quelconque des revendications précédentes, **caractérisé en ce que** les déchets comprennent en outre de l'aluminium et **en ce que** le procédé comprend en outre une étape consistant à récupérer l'aluminium à partir des déchets.

6. Procédé selon l'une quelconque des revendications précédentes, **caractérisé en ce que** les déchets polymères contiennent, dans la fraction polymère, une quantité de composants non polyoléfiniques inférieure à 12 % en poids, et de préférence inférieure à 10 % en poids, par rapport au poids total de la fraction polymère à l'état sec de la matière première constituée par les déchets.

7. Procédé selon l'une quelconque des revendications précédentes, **caractérisé en ce que** les déchets polymères présentent une masse volumique apparente d'au moins 150 g/cm³, de préférence comprise entre 200 et 700 g/cm³, de préférence encore comprise entre 200 et 600 g/cm³, et de manière plus préférentielle comprise entre 250 et 550 g/cm³, déterminée conformément à la norme DIN 53466.

8. Procédé selon l'une quelconque des revendications précédentes, **caractérisé en ce que** le polyéthylène réticulé présent dans les déchets polymères est choisi parmi le groupe constitué du polyéthylène réticulé par peroxyde, du polyéthylène réticulé par silane, du polyéthylène réticulé par irradiation, du polyéthylène réticulé par voie radicalaire, du polyéthylène réticulé par azo, du polyéthylène réticulé par voie radicalaire initiée par UV et de leurs mélanges.

9. Procédé selon l'une quelconque des revendications précédentes, **caractérisé en ce que** les déchets polymères sont obtenus à partir de tuyaux en PEX, de tuyaux en PEX/Al/PEX, de tuyaux en PEX/Al/PE-RT et/ou de tuyaux en PEX/Al/PE.

10. Procédé selon l'une quelconque des revendications précédentes, **caractérisé en ce que** les déchets polymères comprennent ou sont constitués de déchets de tuyaux PEX déchiquetés et d'un flux de déchets plastiques présentant un indice de fluidité à chaud (MFI/21,6) supérieur à 10 g/10 min, la préférence étant donnée à une quantité de flux de déchets plastiques représentant au moins 40 % en poids des déchets, de préférence de 40 à 80 % en poids, par rapport au poids total des déchets.

11. Procédé selon l'une quelconque des revendications précédentes, **caractérisé en ce qu'**il est mis en œuvre dans un réacteur équipé d'un convoyeur à vis et **en ce que** le produit de dépolymérisation gazeux est évacué du réacteur par des unités d'évacuation de gaz disposées le long d'un convoyeur à vis.

12. Procédé selon l'une quelconque des revendications précédentes, **caractérisé en ce que** le temps de séjour des déchets dans le réacteur n'excède pas 60 minutes, de préférence 45 minutes.

13. Procédé selon l'une quelconque des revendications précédentes, **caractérisé en ce que** le réacteur fonctionne à une pression comprise entre 0,7 et 10 bars g.

14. Procédé selon l'une quelconque des revendications précédentes, **caractérisé en ce que** , le produit de dépolymérisation recueilli comprend plus de 30 % en poids, de préférence plus de 50 % en poids et en particulier plus de 60 % en poids, de composants gazeux, par rapport au poids de la fraction polymère des déchets polymères, lesdits composants gazeux comprenant de préférence plus de 50 % en poids d'oléfines, par rapport au poids total des composants gazeux.

15. Procédé selon l'une quelconque des revendications précédentes, **caractérisé en ce que** le produit liquide de dépolymérisation comprend de 35 à 45 % en poids d'une fraction à point d'ébullition élevé, de 40 à 50 % en poids d'une fraction à point d'ébullition moyen et de 15 à 25 % en poids d'une fraction à point d'ébullition bas.
